# EUROPÄISCHE PATENTSCHRIFT

(11) **EP 0 956 281 B1**
(45) Veröffentlichungstag und Bekanntmachung des Hinweises auf die Patenterteilung: **14.08.2002**
(21) Anmeldenummer: 98904048.0
(22) Anmeldetag: 09.01.1998
(51) Int. Cl.: C07C 271/28, A61K 31/27

(54) **NEUE MODIFIKATIONEN DES 2-AMINO-4-(4-FLUORBENZYLAMINO)-1-ETHOXYCARBONYL-AMINOBENZEN SOWIE VERFAHREN ZU IHRER HERSTELLUNG**
NOVEL MODIFICATIONS TO 2-AMINO-4-(4-FLUOROBENZYLAMINO)-1-ETHOXYCARBONYL-AMINOBENZENE AND PROCESSES FOR PREPARING SAID COMPOUND
NOUVELLES MODIFICATIONS DU 2-AMINO-4-(4-FLUOROBENZYLAMINO)-1-ETHOXYCARBONYLE-AMINOBENZENE ET PROCEDES PERMETTANT DE PREPARER LEDIT COMPOSE

(30) Priorität: 20.01.1997 DE 19701694
(43) Veröffentlichungstag der Anmeldung: 17.11.1999
(73) Patentinhaber: VIATRIS GmbH & Co. KG, 60314 Frankfurt am Main (DE)
(72) Erfinder: MEISEL, Peter, D-01097 Dresden (DE); LANDGRAF, Karl-Friedrich, D-01217 Dresden (DE); SCHÄFER, Jürgen, D-01445 Radebeul (DE); THIEL, Wilfried, D-01465 Langebrück (DE); RISCHER, Matthias, D-60388 Frankfurt (DE); OLBRICH, Alfred, D-33790 Halle (DE); KUTSCHER, Bernhard, D-63477 Maintal (DE)
(74) Vertreter: Wibbelmann, Jobst, Dr., Dipl.-Chem.
(86) Internationale Anmeldenummer: EP9800086
(87) Internationale Veröffentlichungsnummer: WO9831663

(56) Entgegenhaltungen:
- DE-A- 4 200 259

## Beschreibung

Die Erfindung betrifft neue Modifikationen der Verbindung 2-Amino-4-(4-fluorbenzylamino)-1-ethoxycarbonyl-aminobenzen der

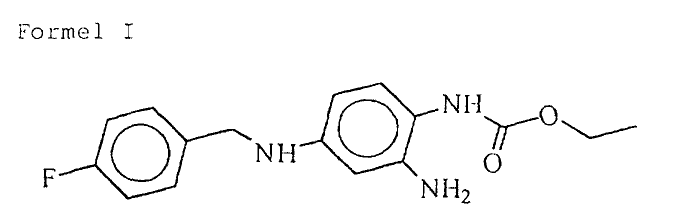

Verfahren zu ihrer Herstellung und ihre Verwendung in pharmazeutischen Zusammensetzungen.

Die Verbindung der Formel I und deren Herstellung wird in dem Patent DE 42 00 259 beschrieben.

Diese Verbindung ist beispielsweise antikonvulsiv, antipyretisch und analgetisch wirksam und kann damit in pharmazeutischen Zubereitungen eingesetzt werden.

Bei der Kristallisation der Verbindung der Formel I werden jedoch hinsichtlich der Kristallgröße und -form zum Teil sehr unterschiedliche Mischprodukte erhalten. Mischungen von Kristallmodifikationen stellen für pharmazeutische Zubereitungen ein großes Problem dar. Insbesondere wirken sich bei Arzneiformen mit hohem Wirkstoffanteil physikalische Inhomogenitäten nachteilig auf die Einhaltung konstanter galenischer Herstellungsbedingungen aus.

Zum anderen können erhebliche Schwankungen in der Stabilität, Reinheit und Uniformität des Fertigproduktes auftreten, so daß den Anforderungen an die pharmazeutische Qualität eines Wirkstoffes nicht genügt werden kann.

Es ist deshalb von großem Interesse, die Verbindung der Formel I in einheitlicher kristalliner Form herzustellen.

Der Erfindung liegt somit die Aufgabe zugrunde, die Verbindung der Formel I in einheitlicher kristalliner Form bereitzustellen, die den pharmazeutischen Erfordernissen entspricht.

Es wurde nun überraschend gefunden, daß die Verbindung der Formel I in 3 verschiedenen reinen Kristall-Modifikationen hergestellt werden kann. Damit können physikalisch homogene Verbindungen der Formel I für die Herstellung pharmazeutischer Fertigprodukte bereitgestellt werden.

Die 3 Modifikationen, A, B und C genannt, weisen unterschiedliche physiko-chemische Eigenschaften auf.

Zur Identifizierung dieser drei Modifikationen der Verbindung der Formel I werden die jeweils charakteristischen Röntgendiffraktogramme herangezogen.

Die Modifikationen unterscheiden sich weiterhin in ihren DSC-Kurven (Differential Scanning Calorimetrie) und zum Teil auch in ihren IR-Spektren sowie durch die jeweils typischen Kristallformen.

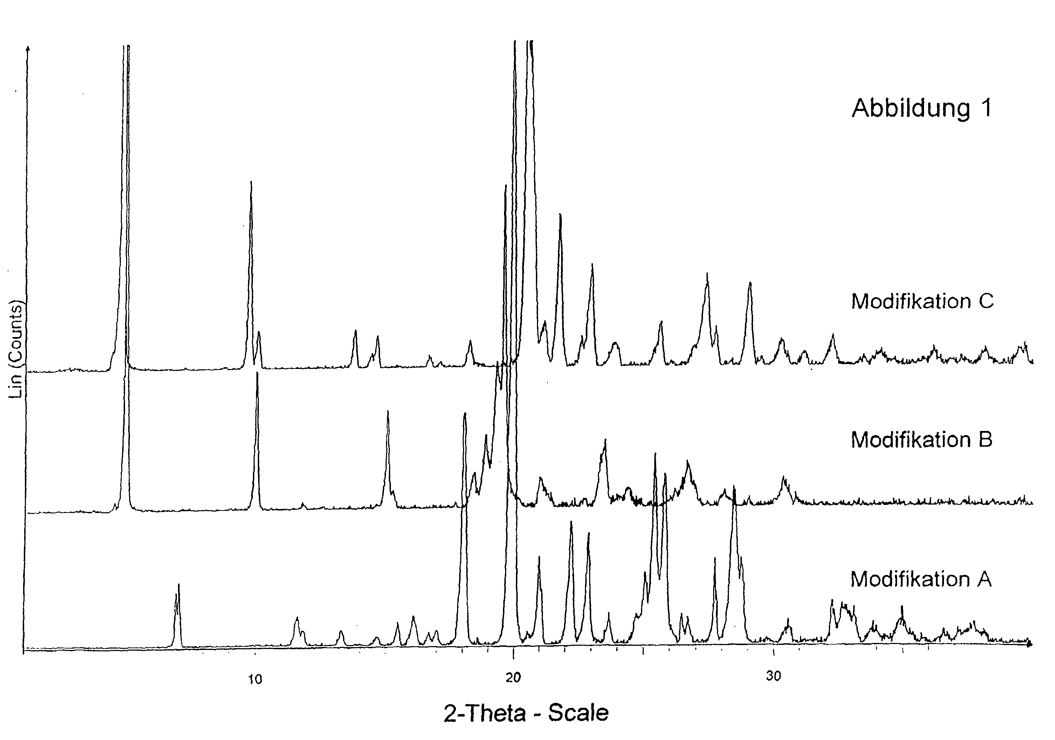

Die Röntgendiffraktogramme gemäß der Abbildung 1 wurden mit einem Pulverdiffraktometer mit CuK_{α}-Strahlung aufgenommen.

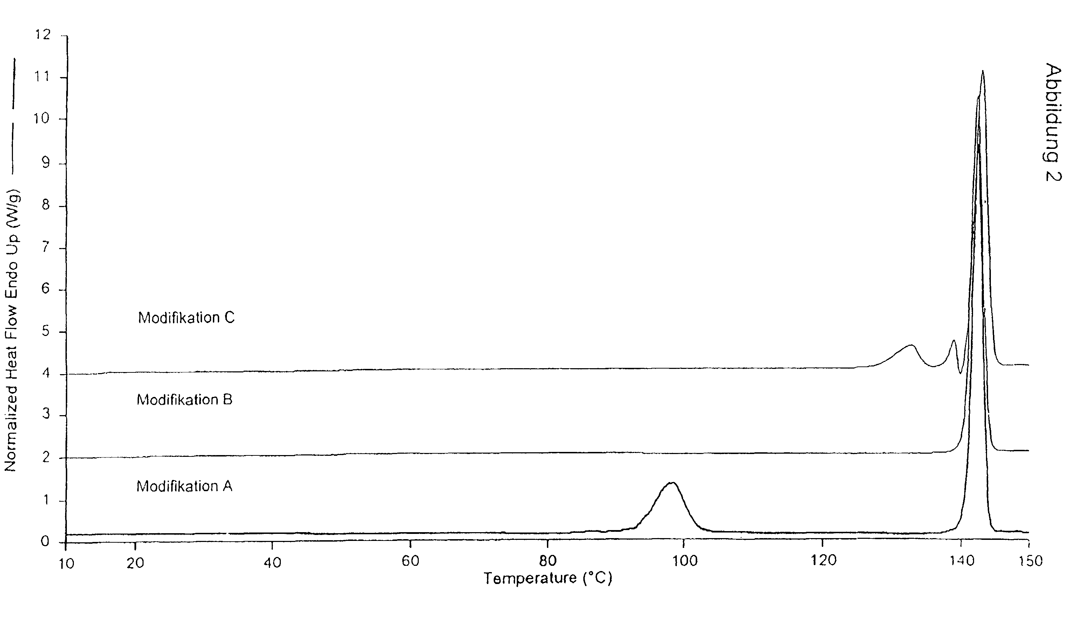

Die Angaben zur DSC-Kurve gemäß Abbildung 2 beziehen sich auf eine Aufheizgeschwindigkeit von 10 K/min. Die angeführten Temperaturen geben jeweils die Lage des Intensitätsmaximums an.

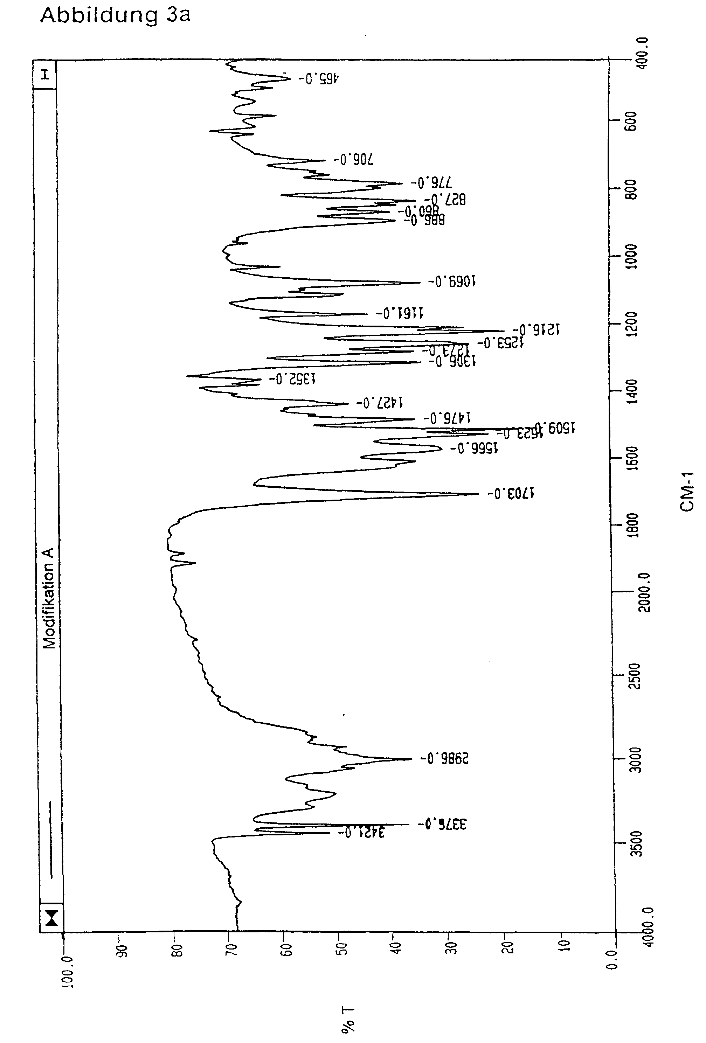

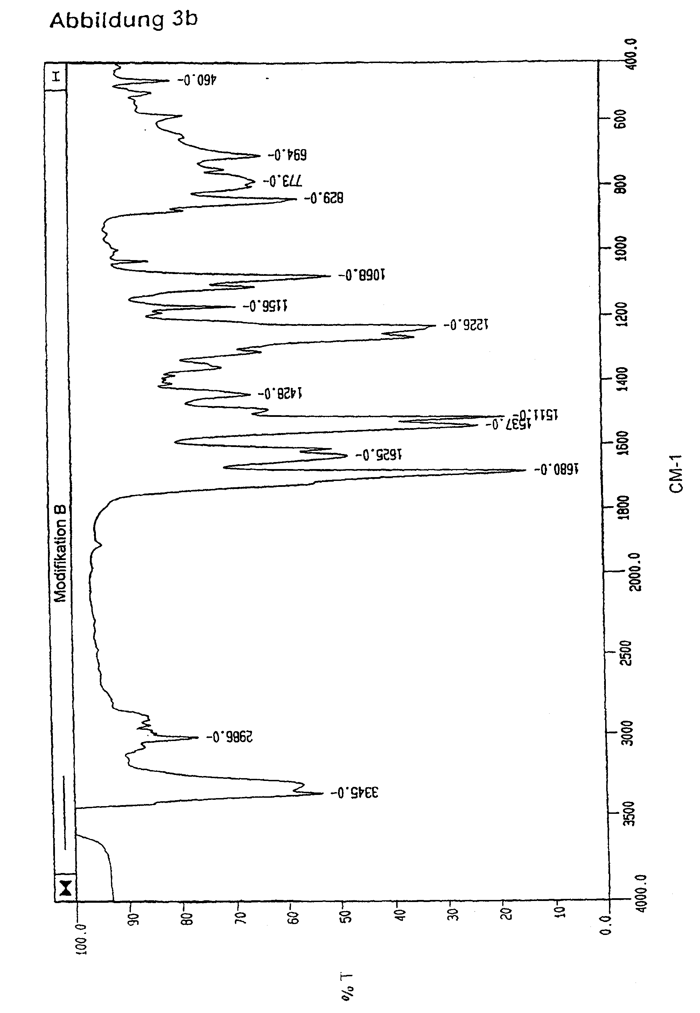

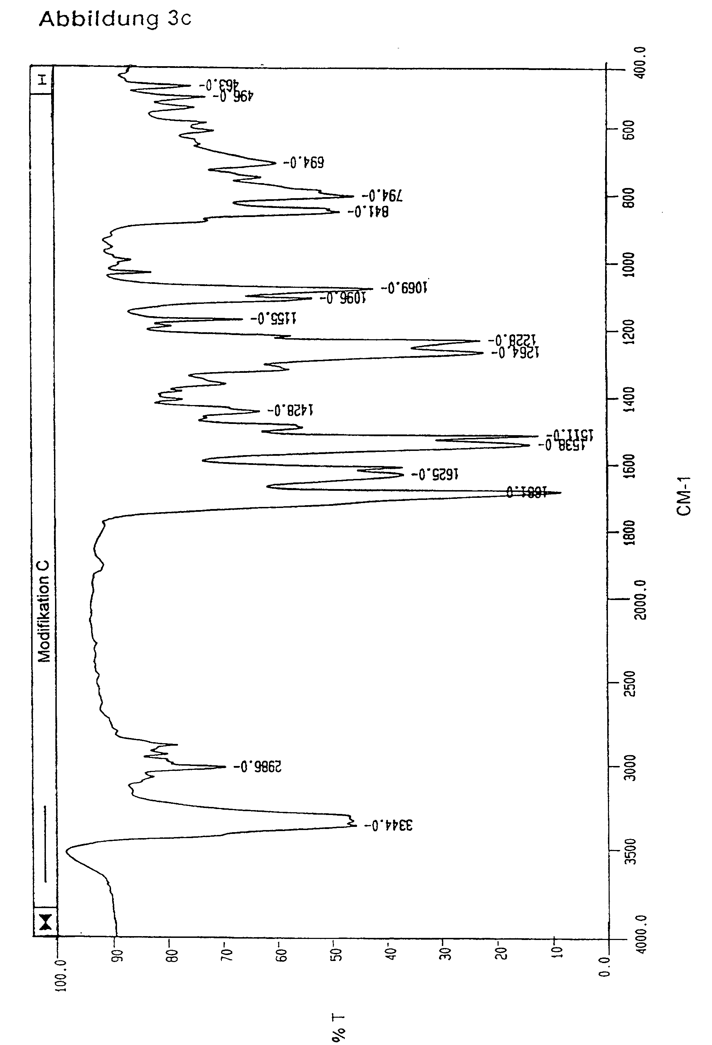

Die abgebildeten IR-Spektren (Abbildung 3a, b, c) wurden an KBr-Preßlingen aufgenommen.

**Die Modifikation A ist charakterisiert durch:**
- das Röntgendiffraktogramm, wobei nicht mit den Reflexen der anderen beiden Modifikationen koinzidierende Reflexe unter anderem bei 6.97°2ϑ (12.67 Å), 18.02°2ϑ (4.92 Å) und 19.94°2ϑ (4.45 Å) beobachtet werden,
- den endothermen A-B-Umwandlungseffekt bei etwa 97°C (Maximum) unterhalb des Schmelzeffektes der Modifikation B bei etwa 142°C in der DSC-Kurve,
- das sich von den anderen beiden Modifikationen durch intensive Schwingungsbanden bei 3421 cm⁻¹ (ν N-H), 3376 cm⁻¹ (ν N-H), 1703 cm⁻¹ (ν C=O) und 886 cm⁻¹ (γ C-H) unterscheidende IR-Spektrum sowie
- vorwiegend nahezu isometrische bis kurzsäulige Kristalle.

**Die Modifikation B ist charakterisiert durch:**
- das Röntgendiffraktogramm, wobei nicht mit den Reflexen der anderen beiden Modifikationen koinzidierende Reflexe unter anderem bei 15.00°2ϑ (5.90 Å), 19.29°2ϑ (4.60 Å) und 19.58°2ϑ (4.53 Å) beobachtet werden,
- das Fehlen thermischer Effekte unterhalb des Schmelzeffektes bei etwa 142°C in der DSC-Kurve und
- vorwiegend länglich tafelige bis stengelige Kristalle.

Die Modifikation C ist charakteristisiert durch:
- das Röntgendiffraktogramm, wobei nicht mit den Reflexen der anderen beiden Modifikationen koinzidierende Reflexe unter anderem bei 9.70°2ϑ (9.11 Å) und 21.74°ϑ (4.09 Å) beobachtet werden,
- zwei mit dem Phasenübergang in die Modifikation B zusammenhängenden endothermen Effekten zwischen etwa 130°C und dem Schmelzeffekt der Modifikation B bei etwa 142°C in der DSC-Kurve und
- vorwiegend tafelige Kristalle.

Die Herstellung der 3 Modifikationen der Verbindung I kann nach folgenden Verfahren erfolgen, wobei die Einhaltung der Bedingungen von besonderer Bedeutung ist.

Die Modifikationen können entweder aus dem Rohprodukt der Verbindung der Formel I oder auch durch Modifikationsumwandlung hergestellt werden.

### Herstellung der Modifikation A:

Die Modifikation A kann durch Rühren in Lösungsmitteln aus den Modifikationen B und C hergestellt werden.

Die Kristallisation der Modifikation A erfolgt vorzugsweise unter Rühren einer übersättigten Lösung der Verbindung I in protischen , dipolar aprotischen oder unpolaren Lösungsmitteln.

Als protische Lösungsmittel können niedere Alkohole, wie Ethanol, 2-Propanol, n-Butanol, als dipolar aprotische Lösungsmittel Acetonitril oder Aceton und als unpolare Lösungsmittel Toluol eingesetzt werden.

Vorzugsweise wird die Kristallisation in Gegenwart von niederen Alkoholen vorgenommen.

Die Kristallisation aus der Lösung erfolgt im Temperaturbereich von -20°C bis 110°C. Insbesondere kann in bestimmten Lösungsmitteln, wie n-Butanol, die Kristallisation der reinen Modifikation A bei Temperaturen bis 110°C erfolgen.
Vorzugsweise wird die reine Modifikation A durch Kristallisation im Temperaturbereich von 20°C bis 50°C erhalten.

### Herstellung der Modifikation B:

Aus einer gesättigten Lösung der Verbindung I erfolgt unter langsamer Abkühlung die Kristallisation der Modifikation B.

Als Lösungsmittel können protische wie Wasser oder unpolare wie Toluol eingesetzt werden.

Vorzugsweise wird die Kristallisation in Gegenwart von Toluol vorgenommen.

Die Kristallisation aus der Lösung kann im Temperaturbereich zwischen 50°C bis 110°C erfolgen, vorzugsweise jedoch zwischen 80°C - 100°C .

Die Modifikation B kann auch durch thermische Phasenumwandlung vorzugsweise aus der Modifikation A bei Temperaturen größer 80°C erhalten werden.

### Herstellung der Modifikation C:

Aus einer gesättigten Lösung der Verbindung I in protischen Lösungsmitteln wie Ethanol und 2-Propanol oder aprotischen Lösungsmitteln wie Toluol kristallisiert unter langsamer Abkühlung bei einer Temperatur von 30°C - 80°C die Modifikation C aus.

Vorzugsweise erfolgt die Kristallisation aus der Lösung bei einer Temperatur von 50°C - 70°C.

Jede dieser Modifikationen der Verbindung I kann zur Verabreichung in galenische Formen verarbeitet werden, die den pharmazeutischen Anforderungen genügen.

Die vorliegende Erfindung betrifft weiter die Verwendung der Modifikationen A, B und C der Verbindung I zur Herstellung von pharmazeutischen Zubereitungen. Sie stellen insbesondere wirkungsvolle Antiepileptika und Neuroprotektiva dar.

Die pharmazeutischen Zubereitungen können im allgemeinen als Einzeldosis zwischen 10 mg bis 200 mg mindestens einer der Modifikationen der Verbindung I enthalten. Bevorzugte Anwendungsformen sind Tabletten.

Die Modifikationen der Verbindung der Formel I können in üblicher Weise mit geeigneten Träger- und/oder Hilfsstoffen zur pharmazeutischen Zubereitung verarbeitet werden.

Insbesondere zeigt die Modifikation A der Verbindung I vorteilhafte Eigenschaften für die galenische Weiterverarbeitung.
- Die Kristallstruktur ist bis etwa 80°C stabil. Auch nach längerer Lagerung bei Temperaturen bis zu 60°C und relativen Luftfeuchtigkeiten bis 70% werden keine Gitterumwandlungen beobachtet.
- Die Modifikation A erfährt bei Kontakt mit Lösungsmitteln wie zum Beispiel Wasser, Ethanol, Aceton oder Toluol keine Gitterumwandlung.
- Die nahezu isometrische bis kurzsäulige Kristallform führt zu einem für die galenische Verarbeitung günstigen körnigen Substanzgefüge.

Die Modifikationen B und C können für spezielle Arzneiformen wie Kapseln und Trockenampullen vorteilhaft eingesetzt werden. So kann beispielsweise die bei der Modifikation C beobachtete bevorzugte Bildung feinkörniger und deshalb besonders schnell löslicher Kristalle Vorteile für die Herstellung von Trockenampullen haben.

Die Herstellungsverfahren für die einzelnen Modifikationen sollen anhand von Beispielen näher erläutert werden:

### Beispiel 1

### Modifikation A

Im 16-1-Lösegefäß werden 2,34 kg der Verbindung I und 0,16 kg Aktivkohle in 7,0 1 Ethanol unter Rühren durch Erwärmen gelöst. Die Lösung wird heiß über einen Druckfilter unter Rühren in ein gekühltes 32-1-Kristallisationsgefäß mit 0,5 1 Ethanol so filtriert, daß die Innentemperatur im Kristallisationsgefäß bei < 45°C gehalten wird. Dann wird mit 0,75 1 heißem Ethanol vom Lösegefäß über den Druckfilter in das Kristallisationsgefäß gespült und die Suspension zügig abgekühlt. Es wird 0,5 Stunden bei 5°C - 12°C nachgerührt und unter Inertbedingungen abgesaugt. Das Produkt wird dreimal mit je 1,2 1 gekühltem Ethanol gewaschen. Anschließend wird das Kristallisat im Vakuumtrockenschrank bei 50°C - 55°C bis zur Massekonstanz getrocknet. Man erhält 2,04 kg (87% der Theorie) der reinen Modifikation A.

### Beispiel 2

### Modifikation A

2 g der Modifikation C werden bei Raumtemperatur in 6 ml Ethanol 2 Tage gerührt. Man erhält quantitativ die Modifikation A.

### Beispiel 3

### Modifikation A

5 g der Modifikation B oder C werden bei Raumtemperatur in 50 ml Toluol 2 Tage gerührt. Man erhält quantitativ die Modifikation A.

### Beispiel 4

### Modifikation A

3 g der Modifikation B werden bei Raumtemperatur in 1,5 ml Aceton 2 Tage gerührt. Man erhält quantitativ die Modifikation A.

### Beispiel 5

### Modifikation A

10 g der Verbindung I werden in 5 ml n-Butanol unter Erwärmen gelöst. Man läßt die Lösung bei 105°C - 110°C kristallisieren, kühlt auf 20°C ab und wäscht die Kristalle nach dem Absaugen mit n-Butanol. Man erhält quantitativ die Modifikation A.

### Beispiel 6

### Modifikation B

10 g der Verbindung I werden mit 20 ml Toluol kurz zum Rückfluß erhitzt und gelöst. Man läßt die Lösung bei 90°C - 100°C kristallisieren saugt ab und wäscht mit 5 ml Toluol. Nach der Trocknung erhält man 9,8 g (98% der Theorie) nadelförmige Kristalle.

### Beispiel 7

### Modifikation B

10 g Substanz der Modifikation A werden 8 Stunden bei 100°C im Trockenschrank gelagert. Man erhält quantitativ die Modifikation B.

### Beispiel 8

### Modifikation C

Im 32-1-Lösegefäß werden 3,0 kg der Verbindung I nach Zusatz von 0,2 kg Aktivkohle in 19,6 1 Isopropanol unter Rühren durch Erwärmen gelöst. Die Lösung wird heiß über einen Druckfilter in ein 32-1-Kristallisationsgefäß so filtriert, daß die Innentemperatur im Kristallisationsgefäß bei 60 - 65°C gehalten wird. Anschließend wird mit 2,5 1 heißem Isopropanol (ca. 70°C) vom Lösegefäß über den Druckfilter in das Kristallisationsgefäß gespült. Nach Kristallisationsbeginn bei 60°C - 65°C wird nachgerührt. Die gebildete Suspension wird zügig abgekühlt, bei 5°C - 12°C nachgerührt und unter Inertbedingungen abgesaugt. Das Kristallisat wird dreimal mit je 2,5 1 gekühltem Isopropanol gewaschen.

Anschließend wird das Kristallisat bei 50°C - 55°C unter Vakuum bis zur Massekonstanz getrocknet. Man erhält 2,64 kg (88% der Theorie) des Wirkstoffes in der Modifikation C.

## Patentansprüche

1. Modifikation A der Verbindung I 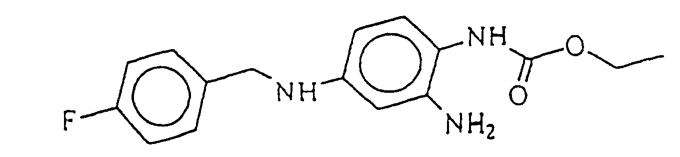 charakterisiert durch das Röntgendiffraktogramm (vergleiche Abbildung 1), wobei nicht mit den Reflexen der anderen beiden Modifikationen koinzidierende Reflexe unter anderem bei 6.97°2ϑ (12.67 Å), 18.02°2ϑ (4.92 Å) und 19.94°2ϑ (4.45 Å) beobachtet werden.

2. Modifikation B der Verbindung I charakterisiert durch das Röntgendiffraktogramm (vergleiche Abbildung 1), wobei nicht mit den Reflexen der anderen beiden Modifikationen koinzidierende Reflexe unter anderem bei 15.00°2ϑ (5.90 Å), 19.29°2ϑ (4.60 Å) und 19.58°2ϑ (4.53 Å) beobachtet werden.

3. Modifikation C der Verbindung I charakterisiert durch das Röntgendiffraktogramm (vergleiche Abbildung 1), wobei nicht mit den Reflexen der anderen beiden Modifikationen koinzidierende Reflexe unter anderem bei 9.70°2ϑ (9.11 Å) und 21.74°2ϑ (4.09 Å) beobachtet werden.

4. Verfahren zur Herstellung der Modifikation A gemäß Anspruch 1, **dadurch gekennzeichnet, dass** man aus einer übersättigten Lösung der Verbindung I in protischen, dipolar aprotischen oder unpolaren Lösungsmitteln bei Temperaturen von -20°C bis 110°C die reine Kristallform auskristallisieren lässt.

5. Verfahren nach Anspruch 4, **dadurch gekennzeichnet, dass** man die Kristallisation bei Temperaturen von 20°C bis 50°C durchführt.

6. Verfahren zur Herstellung der Modifikation A gemäß einem der Ansprüche 4 bis 5, **dadurch gekennzeichnet, dass** als protische Lösungsmittel niedere Alkohole ausgewählt aus Ethanol, 2-Propanol oder n-Butanol, als dipolar aprotische Lösungsmittel Acetonitril oder Aceton und als unpolares Lösungsmittel Toluol eingesetzt werden können.

7. Verfahren nach Anspruch 6, **dadurch gekennzeichnet, dass** niedere Alkohole als Lösungsmittel verwendet werden.

8. Verfahren zur Herstellung der Modifikation A gemäß Anspruch 1, **dadurch gekennzeichnet, dass** Substanz der Modifikationen B und C mit protischen, dipolar aprotischen oder unpolaren Lösungsmitteln bei Raumtemperatur behandelt werden.

9. Verfahren zur Herstellung der Modifikation B gemäß Anspruch 2, **dadurch gekennzeichnet, dass** man aus einer gesättigten Lösung der Verbindung I in protischen oder unpolaren Lösungsmitteln die reine Kristallform bei einer Temperatur von 50°C bis 110°C auskristallisieren lässt.

10. Verfahren zur Herstellung der Modifikation B nach Anspruch 9, **dadurch gekennzeichnet, dass** als protisches Lösungsmittel Wasser und als unpolares Lösungsmittel Toluol eingesetzt wird.

11. Verfahren zur Herstellung der Modifikation B gemäß Anspruch 2, **dadurch gekennzeichnet, dass** man die Modifikation B durch thermische Phasenumwandlung aus der Modifikation A bei Temperaturen größer 80°C herstellt.

12. Verfahren zur Herstellung der Modifikation C gemäß Anspruch 3, **dadurch gekennzeichnet, dass** man aus einer gesättigten Lösung der Verbindung I in protischen oder auch unpolaren Lösungsmitteln die reine Kristallform bei einer Temperatur von 30°C bis 80°C auskristallisieren lässt.

13. Verfahren zur Herstellung der Modifikation C nach Anspruch 12, **dadurch gekennzeichnet, dass** als protische Lösungsmittel Ethanol und 2-Propanol und als unpolares Lösungsmittel Toluol eingesetzt wird.

14. Verfahren zur Herstellung der Modifikation C nach Anspruch 12, **dadurch gekennzeichnet, dass** man die Kristallisation aus der Lösung bei Temperaturen von 50°C bis 70°C durchführt.

15. Verwendung der Modifikation A, B und C der Verbindung I zur Herstellung von pharmazeutischen Zubereitungen.

16. Arzneimittel, enthaltend die Modifikation A, B oder C der Verbindung I und gegebenenfalls Träger und/oder Hilfsstoffe.

## Claims

1. Modification A of the compound I 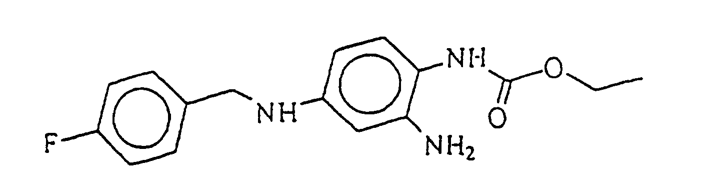 **characterized by** the X-ray diffractogram (see Figure 1), reflections not coinciding with the reflections of the other two modifications being observed, inter alia, at 6.97°2ϑ (12.67 Å), 18.02°2ϑ (4.92 Å) and 19.94°2ϑ (4.45 Å).

2. Modification B of the compound I **characterized by** the X-ray diffractogram (see Figure 1), reflections not coinciding with the reflections of the other two modifications being observed, inter alia, at 15.00°2ϑ (5.90 Å), 19.29°2ϑ (4.60 Å) and 19.58°2ϑ (4.53 Å).

3. Modification C of the compound I **characterized by** the X-ray diffractogram (see Figure 1), reflections not coinciding with the reflections of the other two modifications being observed, inter alia, at 9.70°2ϑ (9.11 Å) and 21.74°2ϑ (4.09 Å).

4. Process for the preparation of the modification A according to Claim 1, **characterized in that** the pure crystal form is allowed to crystallize out at temperatures from -20°C to 110°C of a supersaturated solution of the compound I in protic, dipolar-aprotic or non-polar solvents.

5. Process according to Claim 4, **characterized in that** the crystallization is carried out at temperatures from 20°C to 50°C.

6. Process for the preparation of the modification A according to any of Claims 4 to 5, **characterized in that** as protic solvents lower alcohols selected from ethanol, 2-propanol or n-butanol, as dipolar-aprotic solvents acetonitrile or acetone and as non-polar solvent toluene can be employed.

7. Process according to Claim 6, **characterized in that** lower alcohols are used as solvents.

8. Process for the preparation of the modification A according to Claim 1, **characterized in that** the substance of the modifications B and C are treated with protic, dipolar-aprotic or non-polar solvents at room temperature.

9. Process for the preparation of the modification B according to Claim 2, **characterized in that** the pure crystal form is allowed to crystallize out at a temperature of from 50°C to 110°C from a saturated solution of the compound I in protic or non-polar solvents.

10. Process for the preparation of the modification B according to Claim 9, **characterized in that** the protic solvent employed is water and the non-polar solvent employed is toluene.

11. Process for the preparation of modification B according to Claim 2, **characterized in that** the modification B is prepared from the modification A at temperatures of greater than 80°C by thermal phase conversion.

12. Process for the preparation of the modification C according to Claim 3, **characterized in that** the pure crystal form is allowed to crystallize out at a temperature of from 30°C to 80°C from a saturated solution of the compound I in protic or alternatively non-polar solvents.

13. Process for the preparation of the modification C according to Claim 12, **characterized in that** as the protic solvents ethanol and 2-propanol are employed and as the non-polar solvent toluene is employed.

14. Process for the preparation of the modification C according to Claim 12, **characterized in that** the crystallization from the solution is preferably carried out at temperatures from 50°C to 70°C.

15. Use of the modification A, B and C of the compound I for the production of pharmaceutical preparations.

16. Pharmaceuticals comprising the modification A, B or C of the compound I and, if appropriate, carriers and/or auxiliaries.

## Revendications

1. Modification A du composé I 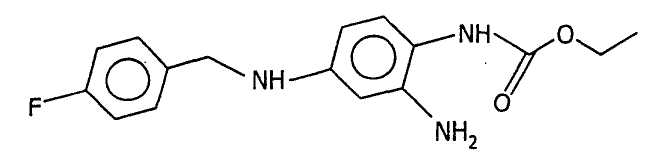 **caractérisée par** le diffractogramme X (voir figure 1) où on observe des réflexions qui ne coïncident pas avec les réflexions des deux autres modifications, entre autres à 6,97°2ϑ (12,67 Å), 18,02°2ϑ (4,92 Å) et 19,94°2ϑ (4,45 Å).

2. Modification B du composé I **caractérisée par** le diffractogramme X (voir figure 1) où on observe des réflexions qui ne coïncident pas avec les réflexions des deux autres modifications, entre autres à 15,00°2ϑ (5,90 Å), 19,29°2ϑ (4,60 Å) et 19,58°2ϑ (4,53 Å).

3. Modification C du composé I **caractérisée par** le diffractogramme X (voir figure 1) où on observe des réflexions qui ne coïncident pas avec les réflexions des deux autres modifications, entre autres à 9,70°2ϑ (9,11 Å) et 21,74°2ϑ (4,09 Å).

4. Procédé de préparation de la modification A selon la revendication 1, **caractérisé en ce que** l'on fait cristalliser la forme cristalline pure dans une solution sursaturée du composé I dans des solvants protiques, aprotiques dipolaires ou non polaires à des températures de -20°C à 110°C.

5. Procédé selon la revendication 4, **caractérisé en ce que** l'on réalise la cristallisation à des températures de 20°C à 50°C.

6. Procédé de préparation de la modification A selon l'une des revendications 4 et 5, **caractérisé en ce que** l'on peut utiliser comme solvants protiques des alcools inférieurs choisis parmi l'éthanol, le 2-propanol et le n-butanol, comme solvants aprotiques dipolaires l'acétonitrile ou l'acétone et comme solvant non polaire le toluène.

7. Procédé selon la revendication 6, **caractérisé en ce que** l'on utilise des alcools inférieurs comme solvant.

8. Procédé de préparation de la modification A selon la revendication 1, **caractérisé en ce que** l'on traite la substance des modifications B et C avec des solvants protiques, aprotiques dipolaires ou non polaires à la température ambiante.

9. Procédé de préparation de la modification B selon la revendication 2, **caractérisé en ce que** l'on fait cristalliser la forme cristalline pure à une température de 50°C à 110°C dans une solution saturée du composé I dans des solvants protiques ou non polaires.

10. Procédé de préparation de la modification B selon la revendication 9, **caractérisé en ce que** l'on utilise comme solvant protique l'eau et comme solvant non polaire le toluène.

11. Procédé de préparation de la modification B selon la revendication 2, **caractérisé en ce que** l'on prépare la modification B par conversion de phase thermique à partir de la modification A à des températures supérieures à 80°C.

12. Procédé de préparation de la modification C selon la revendication 3, **caractérisé en ce que** l'on fait cristalliser la forme cristalline pure à une température de 30°C à 80°C dans une solution saturée du composé I dans des solvants protiques ou encore non polaires.

13. Procédé de préparation de la modification C selon la revendication 12, **caractérisé en ce que** l'on utilise comme solvants protiques l'éthanol et le 2-propanol et comme solvant non polaire le toluène.

14. Procédé de préparation de la modification C selon la revendication 13, **caractérisé en ce que** l'on réalise la cristallisation dans la solution à des températures de 50°C à 70°C.

15. Utilisation des modifications A, B et C du composé I pour la production de préparations pharmaceutiques.

16. Médicament contenant la modification A, B ou C du composé I et éventuellement des supports et/ou adjuvants.
